# EUROPEAN PATENT APPLICATION

(11) **EP 1 422 214 A1**
(43) Date of publication of application: **26.05.2004**
(21) Application number: 02767859.8
(22) Date of filing: 27.08.2002
(51) Int. Cl.: C07C 51/265, C07C 51/43, C07C 51/47, C07C 63/26, B04B 3/04

(54) **METHOD FOR PRODUCING AROMATIC DICARBOXYLIC ACID**

(30) Priority: 29.08.2001 JP 2001259353
(71) Applicant: Mitsubishi Chemical Corporation, Tokyo 108-0014 (JP)
(72) Inventor: ISOGAI, Takayuki Mitsubishi Chemical Corporation, Kitakyushu-shi, Fukuoka 806-0004 (JP); NUMATA, Motoki c/o Mitsubishi Chemical Corporation, Kitakyushu-shi, Fukuoka 806-0004 (JP)
(74) Representative: McCluskie, Gail Wilson
(86) International application number: PCT/JP2002/008606
(87) International publication number: WO 2003/020680

(57) **Abstract**

A process for producing aromatic dicarboxylic acids which comprises subjecting an aromatic compound having an alkyl substituent or a partially oxidized alkyl substituent to liquid-phase oxidation with a molecular oxygen-containing gas in a reaction solvent in the presence of a catalyst, then conducting solid-liquid separation of the formed slurry containing crystals of the produced aromatic dicarboxylic acid, and recovering the crystals, wherein when carrying out solid-liquid separation of the slurry by continuously supplying it to a screen-type centrifugal separator having a screw conveyor disposed therein, a screen with an opening size that allows partial escape of crystals in the supplied slurry through the screen openings is used as the screen of the screen-type centrifugal separator. According to this method, clogging of the centrifugal separator is prevented and solid-liquid separation can be carried out efficiently.

## Description

### Technical Field

The present invention relates to a process for the preparation of aromatic dicarboxylic acids, more particularly to an aromatic dicarboxylic acid preparation process using a screen-type centrifugal separator having a screw conveyor arranged in its inside for the solid-liquid separation of a slurry containing crystals of an aromatic dicarboxylic acid obtained from a reaction.

### Background Art

Aromatic dicarboxylic acids are produced by a process which comprises subjecting an aromatic compound having an alkyl substituent or a partially oxidized alkyl substituent to liquid-phase oxidation with a molecular oxygen-containing gas in a reaction solvent in the presence of a catalyst, then conducting solid-liquid separation of the obtained slurry containing crystals of the produced aromatic dicarboxylic acid, and recovering the crystals.

As a method of the said solid-liquid separation, techniques using a screen-type centrifugal separator having a screw conveyor arranged therein have been proposed (e.g. WO 98/18750).

When carrying out solid-liquid separation by use of a screen, it is important to conduct it efficiently by preventing the screen from getting clogged. Especially in the case of a screen-type centrifugal separator provided with a screw conveyor, clogging is promoted by the cakes formed in a compacted state on the inside of the screen.

Japanese Patent Application Laid-Open (KOKAI) No. 8-294643 proposes a scraper for scratching off the cakes in the screen to maintain the screen function, but this involves the problem that the system is complicated by the provision of the scraper. Also, relating to the size of the screen openings, in for instance "Filtration & Separation (Sep., 2000, p. 245)", it is stated that the size of the screen openings should preferably be set to be 2 to 3 times the grain size of the solid to be separated for preventing the grains from being caught by the screen to cause its clogging. But such a large-mesh screen has the problem of too much escape of the material through the screen openings.

The present invention has been made in view of the above circumstances, and its object is to provide a process for producing aromatic dicarboxylic acids which is improved to make it possible to prevent clogging of the centrifugal separator and to conduct solid-liquid separation efficiently when a slurry containing crystals of an aromatic dicarboxylic acid obtained from a reaction is subjected to solid-liquid separation, by using a screen-type centrifugal separator having a screw conveyor fitted therein. Another object of the present invention is to provide an aromatic dicarboxylic acid preparation process improved so that the crystals recovered by the said solid-liquid separation will be enhanced in purity.

### Brief Description of Drawing

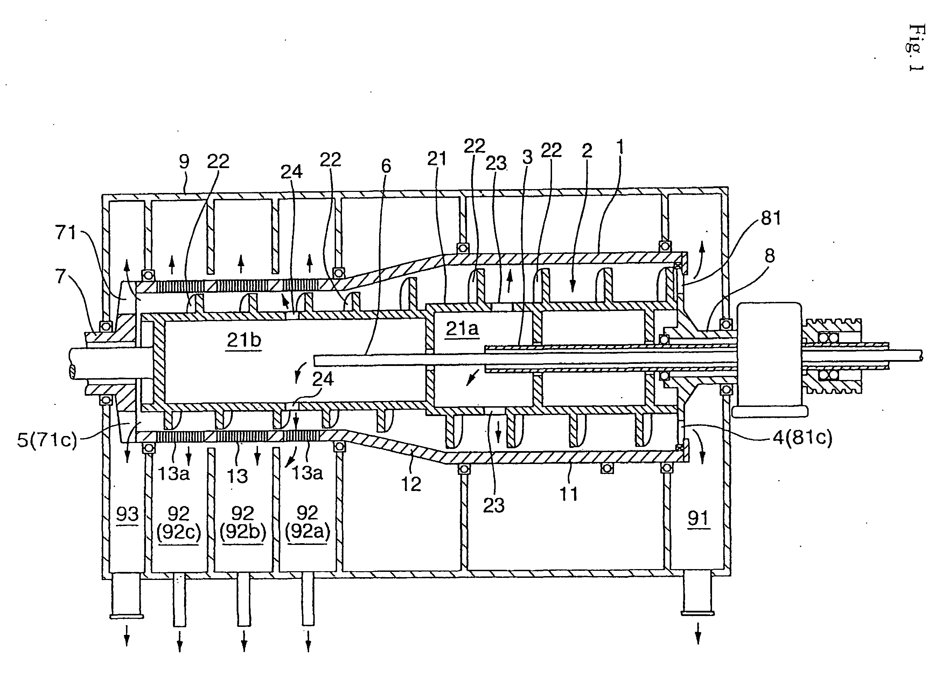

FIG. 1 is a sectional illustration of a screen-type centrifugal separator which is suited for use in the present invention.

### Disclosure of Invention

In order to attain the above objects, the present inventors have pursued studies on the cakes formed by the screw in a compacted state on the inside of the screen, and obtained the following finding. That is, if the grains with indeterminate form and a relatively small diameter in the slurry are allowed to escape through the screen openings, the cakes formed in a compacted state on the inside of the screen by the grains with indeterminate form and a relatively large diameter won't cause no excessive clogging. Also, in case where the said indeterminate form grains are crystals, the purity of the recovered crystals is elevated as the impurities are concentrated in the crystals with a relatively small grain size.

The present invention has been attained on the basis of the above finding. In an aspect of the present invention, there is provided a process for preparing aromatic dicarboxylic acids which comprises subjecting an aromatic compound having an alkyl substituent or a partially oxidized alkyl substituent to liquid-phase oxidation with a molecular oxygen-containing gas in a reaction solvent in the presence of a catalyst, then conducting solid-liquid separation of the formed slurry containing crystals of the produced aromatic dicarboxylic acid, and recovering the crystals, wherein in carrying out solid-liquid separation by continuously supplying the said slurry to a screen-type centrifugal separator having a screw conveyor arranged therein, a screen with an opening size that allows partial escape of crystals in the supplied slurry through the screen openings is used as the screen of the screen-type centrifugal separator.

The present invention is described in detail hereinafter.

First, the liquid-phase oxidation reaction is explained. In the present invention, an aromatic compound having an alkyl substituent or a partially oxidized alkyl substituent is subjected to liquid-phase oxidation with a molecular oxygen-containing gas in a reaction solvent in the presence of a catalyst to obtain a slurry containing an aromatic dicarboxylic acid.

An aromatic compound having an alkyl substituent or a partially oxidized alkyl substituent is used as the reaction material. Such an aromatic compound may be either monocyclic or polycyclic. As the said alkyl substituent, C1 to C4 alkyl groups such as methyl, ethyl, n-propyl and isopropyl can be cited. As the said partially oxidized alkyl substituent, aldehyde group, acyl group, carboxyl group and hydroxyalkyl group can be mentioned as examples.

As typical examples of the aromatic compounds having an alkyl substituent (alkyl-substituted aromatic hydrocarbons), dialkylbenzenes such as m-diisopropylbenzene, p-diisopropylbenzene, m-cymene, p-cymene, m-xylene and p-xylene, dialkylnaphthalenes such as dimethylnaphthalenes, diethylnaphthalenes and isopropylnaphthalenes, and dialkylbiphenyls such as dimethylbiphenyls can be cited.

As typical examples of the aromatic compounds having a partially oxidized alkyl substituent, 3-methylbenzaldehyde, 4-methylbenzaldehyde, m-toluic acid, p-toluic acid, 3-formylbenzoic acid, 4-formylbenzoic acid, and formylnaphthalenecarboxylic acids can be cited.

As the catalyst, combinations of heavy metal compounds and bromine compounds can be used. As the heavy metal in the heavy metal compounds, cobalt, manganese, nickel, chromium, zirconium, copper, lead, hafnium and cerium can be mentioned for instance. As the compounds of heavy metals, acetates, nitrates, acetylacetates, naphtenates, stearates and bromides can be mentioned for instance. As the bromine compounds, inorganic bromine compounds such as molecular bromine, hydrogen bromide, sodium bromide, potassium bromide, cobalt bromide and manganese bromide, and organic bromine compounds such as methyl bromide, methylene bromide, bromoform, benzyl bromide, bromomethyltouene, dibromoethane, tribromoethane and tetrabromoethane can be cited.

The ratio of the bromine compound to the heavy metal compound is selected from the range of usually from 0.05 to 10 moles, preferably from 0.1 to 2 moles, as expressed in terms of the ratio of the bromine atoms to one mole of the heavy metal atoms. The amount of the catalyst to be used is selected from the range of usually from 10 to 10,000 ppm, preferably from 100 to 5,000 ppm, as expressed in terms of concentration of the heavy metal in the reaction solvent.

As the reaction solvent, lower aliphatic carboxylic acids are preferably used. Typical examples of such lower aliphatic carboxylic acids are acetic acid, propionic acid and butyric acid. The lower aliphatic carboxylic acids can be used alone or as a mixture with water. The amount of the reaction solvent to be used is selected from the range of usually from 0.5 to 70 parts by weight, preferably from 2 to 50 parts by weight as expressed in terms of the ratio to one part by weight of the reaction material in the liquid phase section.

As the molecular oxygen-containing gas, oxygen and air can be mentioned for instance. The molecular oxygen-containing gas is supplied in excess of the quantity necessary for oxidizing the aromatic compound into an aromatic carboxylic acid. In case of using air, its amount to be used is selected from the range of usually from 2 to 20 Nm³, preferably from 2.5 to 15 Nm³ as expressed in terms of the ratio to 1 kg of the aromatic compound used as oxidation material.

Usually, the reaction is carried out at normal temperature, and the reaction time is selected from the range of usually from 4 to 180 minutes, preferably from 6 to 120 minutes. Here, in case where the oxidation reaction is carried out continuously, the said reaction time is residence time. The aromatic dicarboxylic acid produced by the reaction is partly precipitated as crystals, and a slurry is formed with another part of the produced aromatic dicarboxylic acid being dissolved in the solvent. For instance, in case of producing terephthalic acid by liquid-phase oxidation of p-xylene the slurry is usually composed of acetic acid as reaction solvent, terephthalic acid crystals, catalyst dissolved in the reaction solvent, unreacted material, by-products and unprecipitated terephthalic acid. The average grain size of the terephthalic acid crystals is usually 120 µm ± 40 µm.

Next, solid-liquid separation is explained. In the present invention, the slurry containing the crystals of the aromatic dicarboxylic acid produced from the above reaction is subjected to solid-liquid separation and the crystals are recovered. Thus, the crude crystals recovered from the reaction step are usually treated in the purifying step, and by solid-liquid separation of the slurry formed in the purification step, a high-purity aromatic dicarboxylic acid is recovered. Also, cleaning of the crystals can be performed during solid-liquid separation. Lower aliphatic acids such as acetic acid are favorably used as the cleaning fluid.

The said solid-liquid separation is carried out using a screen-type centrifugal separator equipped with a screw conveyor in the inside. In the present invention, it is possible to use the screen-type centrifugal separators of various structures, no matter what their appellations may be, as far as they are a separator capable of solid-liquid separation on a screen by the action of a centrifugal force while transporting the material to be treated by a screw conveyor. For example, "decanter-type centrifugal separator" (Japanese Patent Application Laid-Open (KOKAI) No. 7-155643, etc.) and "screen ball-type decanter centrifugal separator" (Japanese Patent Application Laid-Open (KOKAI) No. 2000-350946, WO98/18750, etc.) are known.

The screen-type centrifugal separator shown in FIG. 1 is of a structure having an outer rotating cylinder (1), a screw conveyor (2) comprising a cylindrical rotating shaft (21) and a screw (22) and designed to be capable of relative rotation in the said outer rotating cylinder, and a slurry supply pipe (3) disposed inside of the rotating shaft of the said screw conveyor to supply a slurry to the inside of the said shaft. In the proximal end side of the screw conveyor (2) is provided a slurry supply port (23) for supplying the slurry to the outer rotating cylinder (1). The outer rotating cylinder (1) is constituted from a large-diameter portion (11) on the proximal end side, a slant portion (12) gradually lessened in diameter, and a small-diameter portion (13) where a screen (13a) is mounted. An overflow port (4) is provided at the proximal end of the large-diameter portion (11), and a solid discharge port (5) is provided at the fore-end of the small-diameter portion (13).

Further, in a preferred embodiment of the invention, the inside of the rotating shaft (21) of the screw conveyor is partitioned into a slurry supply portion (21a) on the proximal end side and a cleaning fluid supply portion (21b) on the fore-end side. The slurry supply portion (21a) is designed to be capable of supplying the slurry from the slurry supply pipe (3), and the cleaning fluid supply portion (21b) is designed to be capable of supplying the cleaning fluid from the cleaning fluid supply pipe (6) inserted into the inside of the slurry supply portion (3). At a part on the fore-end side of the screw conveyor (2) is provided a cleaning fluid supply port (24) for supplying a cleaning fluid to the small-diameter portion (13) of the outer rotating cylinder (1).

Openings (71c) are provided in the outer peripheral portion of the flange (71) of the outer rotating cylinder drive shaft (7), and these openings constitute the solid discharge port (5). Also, openings (81c) are provided in the outer peripheral portion of the flange (81) of the screw conveyor drive shaft (7) to constitute the overflow port (4).

All of the said elements are housed in a casing (9). The inside of casing (9) is divided into an overflowed liquid reservoir (91), a filtrate reservoir (92) and a solid reservoir (93) by partition walls, and the filtrate reservoir (92) is further divided into three compartments (92a), (92b) and (92c) by partition walls along the longitudinal direction of the small-diameter portion (13) for the convenience of filtrate sampling. Discharge piping is provided for each of the said overflowed liquid reservoir (91), compartments (92a), (92b) and (92c) of filtrate reservoir (92), and solid reservoir (93).

An outstanding feature of the present invention resides in that a screen with an opening size that allows partial escape of the crystals in the supplied slurry through the screen openings is used as the screen of the screen-type centrifugal separator. Here, "partial escape of the crystals through the screen openings" means that there takes place escape of the crystals of an amount equivalent to 1% by weight or more through the screen openings.

Thus, the opening size of the screen used in the present invention must be the one that can cause continuous escape of the crystals of an amount equivalent to 0.01A to 0.1A (kg/hr) where A (kg/hr) is the rate of the crystals in the continuously supplied slurry. It is considered that escape through the screen openings occurs preferentially with the crystals of small grain diameters, so that in the present invention, such escape through the screen openings is initiated with the crystals of relatively small diameters in the slurry.

The opening size is defined as follows. That is, when the average grain size of the crystals in the slurry is B (µm), the opening size of the screen is usually not less than (B - 10) µm and not more than (B + 80) µm, preferably not less than (B - 5) µm and not more than (B + 60) µm. Therefore, in case where the average grain size of the crystals is 100 µm, the opening size of the screen is usually 90 to 180 µm, preferably 95 to 160 µm.

The opening size generally represents the minimal width of the opening and can be determined according to the shape of the opening of the screen in the following way. That is, the opening size is the width of the opening when it is slit-shaped, the diameter when the opening is circular, the minor diameter when the opening is elliptic, the length of the shorter side of the opening when it is rectangular, the length of one side of the opening when it square, the shortest distance between the two parallel sides when the opening is rhombic, and the shortest distance between the longer sides of the opening when it is parallelogramic with other configurations.

The grain size of crystals in the slurry can be measured by the various methods commonly known for the measurement of grain size of powdery materials, but in the present invention, the values determined by a laser scatter type grain-size distribution meter are used.

As a consequence of the above mechanism, in the case of the present invention, the cakes of the crystals formed in a compacted state in the inside of the screen, more definitely in the space between the outer rotating cylinder (1) and the screw (22), are composed of the crystals with relatively large grain sizes. The reason why such cakes don't cause excessive clogging has not yet been clarified definitely but may be accounted for as follows.

That is, generally, in case where the grains of indeterminate form with relatively small sizes are contained in the cakes, such cakes become firm and solid due to a strong bridging action of the grains. On the other hand, the cakes composed of the grains of indeterminate form with relatively large sizes tend to collapse easily because of the absence of the strong bridging action. Therefore, it is supposed that the said cakes in the present invention keep on collapsing and are incessantly renewed, and this is considered the reason why the said cakes cause no excessive clogging of the screen.

When using a screen with an opening size that will make the escape rate less than 1% by weight, the desired clogging preventive effect can not be achieved. On the other hand, in case of using a screen with a too large opening size, the anti-clogging effect may reach a saturation while the escape rate becomes too high, making the operation inefficient. Therefore, the escape rate is preferably not higher than 40% by weight, more preferably not higher than 20% by weight, still more preferably not higher than 10% by weight.

Also, since the impurities are concentrated in the crystals of relatively small grain sizes, purity of the recovered crystals is hiked by the said escape. That is, in the case of preparation of terephthalic acid by liquid-phase oxidation of p-xylene the recovered crystals are low in concentration of paratoluic acid which is a main impurity in the crystals.

In the present invention, solid-liquid separation is performed in the following way. Slurry is supplied to the large-diameter portion (11) from the slurry supply pipe (3) through the slurry supply portion (21a), and here, the slurry is subjected to a centrifugal separatory action of the outer cylinder (1) rotating at high speed to undergo solid-liquid separation.

The separated liquid is taken out from the overflow port (4) through the overflowed liquid reservoir (91). In the meanwhile, the crystals are transferred from the slant portion (12) to the small-diameter portion (13) by the operation of screw (22). Mother liquor in the crystals is also separated by the centrifugal force on the screen (13a). At the same time, the cleaning fluid is supplied from the cleaning fluid supply pipe (6). This cleaning fluid is sprayed to the transferred crystals from the cleaning fluid supply port (24) through the cleaning fluid supply portion (21b). The cleaned and dehydrated crystals are discharged from the solid discharge port (5) through the solid reservoir (93).

The solid-liquid separating conditions are not specifically defined, but it is preferable to exert a centrifugal force of 300 to 5,000 G on the screen and to set the residence time for the solid-liquid separation on the screen at 2 to 20 seconds. The said residence time is defined to designate the residence time after the slurry has passed the cleaning fluid supply area in case where a cleaning fluid supply port is provided.

When the centrifugal force is less than 300 G, the residence time for solid-liquid separation on the screen becomes too long, and when the centrifugal force exceeds 5,000 G, the safe operation of the centrifugal separator is jeopardized. The preferred range of centrifugal force is 500 to 3,000 G. Adjustment of such a centrifugal force is made by controlling the revolutions of the drive motor of the outer rotating cylinder (1).

When the residence time is less than 2 seconds, it is unable to effect sufficient solid-liquid separation, and when the residence time exceeds 20 seconds, the solid-liquid separating effect saturates, resulting in a reduced operating efficiency of the centrifugal separator. The preferred residence time is 3 to 5 seconds. Adjustment of such a residence time can be made either by a design method involving the size (length) of the screen or by control of an operating condition - difference in rotational speed between the outer rotating cylinder (1) and the screw conveyor (2) (namely, transfer speed of the material to be treated on the screen).

### Best Mode for Carrying out the invention

Hereinafter, the present invention is described in further detail with reference to the examples thereof, but it should be understood that the present invention is not limited to these examples but can be embodied in other forms within the purview of the invention.

### Examples 1 and 2 and Comparative Example 1

A slurry containing crystals of terephthalic acid (solid concentration: 30 wt%; average grain size: 100 µm; paratoluic acid (P-TA) concentration in the solid: 180 ppm) was supplied continuously at 40°C and under normal pressure to a screen-type centrifugal separator shown in FIG. 1 having a screen (slit type) with an opening size shown in Table 1, and the slurry was subjected to solid-liquid separation under the conditions shown in Table 1. The centrifugal force in Table 1 shows the values on the screen. Results are shown in Table 1.

The average grain size of terephthalic acid crystals in the table is the value of grain size at 50% integration read from the distribution of grain size measured by a grain size distribution meter ("LASER MICRON SIZER LMS-24") mfd. by Seishin Enterprise Co., Ltd. The screen opening size was determined as the average value by magnifying the slit portion by a microscope and measuring width of the openings at 360 points.

### Example 3

Solid-liquid separation was conducted in the same manner as in Example 1 except for use of a slurry containing terephthalic acid crystals with an average grain size of 110 µm and having a solid concentration of 30% by weight, use of a screen (slit type) with an opening size of 150 µm, and employment of the conditions of 160°C and 0.65 MPa. Results are shown in Table 1.

**Table 1**

| | Example 1 | Example 2 | Comp. Example 1 | Example 3 |
|---|---|---|---|---|
| Screen opening size (µm) | 100 | 100 | 70 | 150 |
| Slurry throughput (t/hr) | 1.1 | 1.5 | 1.1 | 6.0 |
| Centrifugal force (G) | 750 | 1000 | 750 | 750 |
| Ratio of crystals which escaped through screen openings (wt%) | 3.0 | 5.2 | 0.5 | 5.9 |
| Average grain size of crystals which escaped through screen openings (µm) | 60 | 64 | 15 | 110 |
| Recovered crystals | | | | |
| · Liquid content (wt%) | 11 | 10 | 20 | 11 |
| · P-TA concentration (ppm) | - | 157 | - | - |

As is apparent from the results shown in Table 1, in the case of Examples 1 to 3, the liquid content in the recovered solid matter is lower than in Comparative Example 1. This is attributable to the prevention of clogging to allow effective removal of the liquid.

### Referential Examples 1 to 6

In order to ensure the effect of the residence time, solid-liquid separation was conducted in the same manner as in Example 1 by employing the conditions shown in Table 2 (the centrifugal force on the screen being 750 G). The liquid content of the recovered cakes is shown in Table 2.

**Table 2**

| Referential Example | Residence time (sec) | Slurry supply rate (kg/hr) | Liquid content of recovered cakes (wt%) |
|---|---|---|---|
| 1 | 3.2 | 1110 | 13.0 |
| 2 | 3.2 | 1480 | 12.7 |
| 3 | 5.6 | 1110 | 11.6 |
| 4 | 5.6 | 1480 | 11.1 |
| 5 | 0.8 | 1110 | 19.4 |
| 6 | 0.8 | 1480 | 21.4 |

The residence time in Table 2 is the time after the slurry has passed the supply area of the cleaning fluid spurted from the cleaning fluid supply port (24) (more specifically, the time after the point of time at which 2 seconds have passed after the slurry reached just below the cleaning fluid supply port (24)). The residence time of each case and the liquid content of the recovered cakes were determined in the following way for convenience. That is, the filtrate specimen was recovered from the position corresponding to the particular residence time shown above, then the solid concentration in the filtrate specimen in the residence time was measured, and the liquid content of the recovered cakes was determined from the material balance calculations.

As is apparent from the results shown in Table 2, in the case of Referential Examples 1 to 4, the operation of the centrifugal separator is conducted efficiently and the cakes well reduced in liquid content are recovered because of appropriate residence time. In the case of Referential Examples 5 and 6, on the other hand, the recovered cakes are high in liquid content due to short residence time.

### Industrial Applicability

According to the present invention, clogging of the centrifugal separator is prevented when a slurry containing crystals of an aromatic dicarboxylic acid obtained from a reaction is subjected to solid-liquid separation. Also, according to the present invention, the purity of crystals is enhanced. Therefore, the industrial value of the present invention is high.

## Claims

1. A process for preparing aromatic dicarboxylic acids which comprises subjecting an aromatic compound having an alkyl substituent or a partially oxidized alkyl substituent to liquid-phase oxidation with a molecular oxygen-containing gas in a reaction solvent in the presence of a catalyst, then conducting solid-liquid separation of the formed slurry containing crystals of the produced aromatic dicarboxylic acid, and recovering the crystals, wherein in carrying out solid-liquid separation by continuously supplying the said slurry to a screen-type centrifugal separator having a screw conveyor arranged therein, a screen with an opening size that allows partial escape of crystals in the supplied slurry through the screen openings is used as the screen of the screen-type centrifugal separator.

2. The process according to claim 1 wherein a screen with an opening size that allows escape of an amount equivalent to 1 to 10% by weight of crystals in the supplied slurry is used as the screen of the screen-type centrifugal separator.

3. The process according to claim 1 using a screen-type centrifugal separator having an outer rotating cylinder (1), a screw conveyor (2) comprising a cylindrical rotating shaft (21) and a screw (22) and arranged to be capable of relative rotation in said outer rotating cylinder, and a slurry supply pipe (3) disposed in the inside of the rotating shaft of said screw conveyor and adapted to supply the slurry to the inside of said rotating shaft, said screw conveyor (2) having provided at a part on its proximal end side a slurry supply port (23) for supplying the slurry to the outer rotating cylinder (1), said outer rotating cylinder (1) consisting of a large-diameter portion (11) on the proximal end side, a slant portion (12) gradually reduced in diameter, and a small-diameter portion (13) provided with a screen (13a), said large-diameter portion (11) having formed at its proximal end an overflow port (4), and said small-diameter portion (13) having at its fore end a solid discharge port (5).

4. The process according to claim 3 wherein the inside of rotating shaft (21) of the screw conveyor is partitioned into a slurry supply portion (21a) on the proximal end side and a cleaning fluid supply portion (21b) on the frontal end side, said slurry supply portion (21a) being designed to be capable of supplying the slurry through slurry supply pipe (3), said cleaning fluid supply portion (21b) being designed to be capable of supplying a cleaning fluid to the inside of slurry supply pipe (3) through a cleaning fluid supply pipe (6), and said screw conveyor (2) having provided at a part on its frontal end side a cleaning fluid supply port (24) for supplying the cleaning fluid to the small-diameter portion (13) of the outer rotating cylinder (1).

5. The process according to claim 1 wherein the residence time for the solid-liquid separation on the screen is set to be 2 to 20 seconds, provided that in case where a cleaning fluid supply port is provided, the residence time is the time after the slurry has passed the cleaning fluid supplied area, and a centrifugal force of 300 to 5,000 G is exerted on the screen.

6. The process according to claim 1 wherein the average grain size of crystals in the slurry is 80 to 160 µm.

7. The process according to claim 6 wherein when the average grain size of crystals in the slurry is B (µm), the opening size of the screen is not less than (B - 10) µm and not more than (B + 80) µm.
